# EUROPÄISCHE PATENTANMELDUNG

(11) **EP 0 797 990 A2**
(43) Veröffentlichungstag der Anmeldung: **01.10.1997**
(21) Anmeldenummer: 97100386.8
(22) Anmeldetag: 30.03.1994
(51) Int. Cl.: A61K 31/135

(54) **Verwendung von Steroid-Antogonisten zur Therapie und Prophylaxe von Demenz-Erkrankungen**

(30) Priorität: 10.04.1993 DE 4311870
(62) Teilanmeldung aus: 94912437.4
(71) Anmelder: Altramed Holdings Ltd., Hamilton HM11 (BM)
(72) Erfinder: Denecke, Rainer Dr., 20148 Hamburg (DE)
(74) Vertreter: Klickow, Hans-Henning

(57) **Zusammenfassung**

Das Präparat dient zur Therapie und/oder Prophylaxe von Demenz-Erkrankungen, insbesondere von Erkrankungen infolge regressiver Zellveränderungen. Als Wirkstoff ist eine Dosis mindestens eines Steroid-Antagonisten enthalten. Es ist auch eine Mischung derartiger Substanzen verwendbar.

## Beschreibung

Die Erfindung betrifft ein Präparat zur Therapie und Prophylaxe von Demenz-Erkrankungen, insbesondere von Erkrankungen infolge regressiver Zellveränderungen.

Das Auftreten derartiger Erkrankungen wird mit steigendem Lebensalter wahrscheinlicher. Dies hat zur Folge, daß mit steigender Lebenserwartung und einem hieraus resultierenden wachsenden Anteil älterer Menschen an der Bevölkerung die Prävalenz altersbedingter Demenz-Erkrankungen permanent zunimmt. Alterungserscheinungen im Bereich des zentralen Nervensystems werden von einer Vielzahl struktureller und funktioneller Alterationen begleitet. Dies sind beispielsweise Hirnschrumpfung und Gehirngewichtsverlust, Nervenzelldegenerationen , diverse Amyloidformationen und Läsionen des zerebralen Gefäß-Bindegewebs-Apparates.

Die oben genannten Alterationen betreffen insbesondere Personen, die im weiteren Sinne von der Alzheimer-Krankheit betroffen sind. Dies bedeutet, daß zum einen bei einer vorhandenen Alzheimer-Krankheit die oben genannten Alterationen besonders intensiv auftreten, zum anderen können die oben genannten Alterationen die Symptomatik der Alzheimer-Krankheit verstärken oder deren Auftreten begünstigen. Läsionen des cerebralen Gefäß-Bindegewebs-Apparates sind z.B. die Ursachen der Multi-Infarkt-Demenz (MID). Über die Ursachen der Multiplen Sklerose (MS) wird gegenwärtig noch heftig diskutiert.

Als weiteres neurologisches Leiden ist Morbus-Parkinson zu nennen. Diese Erkrankung tritt zusammen mit einem ausgeprägten Dopaminmangel im Bereich der Substantia nigra und im Striatum auf. Ursache dieser Erkrankung ist eine Degeneration von dopaminergen nigrostriären Projektionsneuronen. Auch bei dieser Krankheit wurden Demenzerscheinungen beschrieben.

Neben den bereits erwähnten Demenz-Erkrankungen treten aber auch eine Vielzahl weiterer allgemeiner Ab nutzungserkrankungen auf; z.B. "impaired brain function of old age" (IBFO). Die Wahrscheinlichkeit des Auftretens von Schlaganfällen wird ebenfalls mit zunehmendem Alter größer. Weiterhin sind Krankheiten auf der Basis arteriosklerotischer Gefäßveränderungen zu nennen.

Ein weiterer Effekt, der mit zunehmendem Alterungsprozeß stärker auftritt, ist eine verminderte Adaptionsfähigkeit des "Streß-Managing-Systems". Dieses körpereigene System zur Streßbewältigung ist im Bereich Hypothalamus-Hypohyse-Nebennieren angesiedelt. Die Wirkungsweise dieses Streßbewältigungssystems beruht im wesentlichen darauf, daß der menschliche Körper bei einem Auftreten von Verletzungen, Infektionen oder psychosozialen Streß zum Beispiel mit der Freisetzung einer Vielzahl von Mediatoren reagiert, die aufeinander fein abgestimmt die Homöostase wiederherstellen.

Die Mediatoren sorgen u.a. dafür, daß eine gesteigerte Zellaktivität wieder reduziert wird, um eine Dauerüberlastung der Zellen zu vermeiden, die zu Zellschäden und im Extremfall zum Absterben der Zellen führen könnte.

Ein Beispiel für derartige Mediatoren sind die Steroid-Hormone. Diese Steroid-Hormone binden spezifisch an Rezeptoren, die im Bereich des Zellkernes angeordnet sind. Durch diese Anbindung an die Rezeptoren werden ein breites Spektrum von metabolischen Prozessen induziert. Darüber hinaus treten eine Reihe von neuroendokrino-immunologischen Interaktivitäten auf, die die Integrität und die Gesundheit des betroffenen Individuums sicherstellen.

Bislang ist kein Präparat bekannt, das mit geringen Nebenwirkungen einem Auftreten von altersbedingten Abnutzungserscheinungen vorbeugen kann bzw. bei einer bereits aufgetretenen Erkrankung einem Fortschreiten der Erkrankung entgegenwirkt bzw. die Erkrankung therapiert.

Aufgabe der vorliegenden Erfindung ist es daher, ein Präparat der einleitend genannten Art derart anzugeben, daß bei der Therapie und Prophylaxe von Demenz-Erkrankungen eine hohe Wirksamkeit bei geringen Nebenwirkungen erreicht wird.

Diese Aufgabe wird erfindungsgemäß dadurch gelöst, daß als Wirkstoff eine Dosis mindestens eines Steroid-Antagonisten enthalten ist.

Durch das Einbringen von Steroid-Antagonisten als Zellkernrezeptoren-Blocker oder als Modulatoren in der Steroid-Rezeptor-Superfamilie werden Mechanismen aktiviert, die interaktive Beziehungen zwischen offensichtlich nicht verwandten Systemen induzieren. Durch diese Induktion ist es möglich, stimulierende Effekte auf ein gealtertes oder alteriertes Neuroendokrinium zu erzielen. Hieraus resultiert die Möglichkeit einer Modulation und Therapie von Nervensystemläsionen.

Es können beispielsweise pure oder partielle Steroid Antagonisten bzw. Agonisten verwendet werden. Ein derartiger partieller Steroid-Antagonist ist beispielsweise Tamoxifen. Tamoxifen hat zusätzlich eine inhibitorische Wirkung auf L- und N-Typ Ca²⁺-Kanäle. Durch einen derartigen Kalzium-Antagonismus können zu hohe intrazelluläre Ca²⁺-Spiegel verhindert werden, die einen Zelltod zur Folge haben könnten. Dies bedeutet, daß Tamoxifen neuroprotektiv wirkt.

Darüber hinaus ist auch ein Einfluß von Tamoxifen auf CA²⁺-aktivierte K⁺-Kanäle festzustellen. Bezüglich dieser Kanäle liegen Indizien vor, daß mit einem Einfluß auf Lern- und Gedächtnisleistungen zu rechnen ist. Ein Ligand wie Tamoxifen, der auf beide Kanaltypen wirkt, kann somit akut Lern- und Gedächtnisleistungen positiv beeinflussen und chronisch antidegenerativ wirken. Es handelt sich somit bei einer derartigen Substanz um ein ideales Mittel gegen die Alzheimer-Demenz. Dieses Wirkungsprinzip von Tamoxifen ist auch bei anderen Steroid-Antagonisten festzustellen. Von Antiöstrogenen ist bekannt, daß sie antioxidative Eigenschaften besitzen. Sie vermindern die Membranfluidität und stabilisieren somit Zellmembranen gegen Lipidperoxidation.

Die grundsätzliche Wirkung von Antiöstrogenen resultiert also sowohl daraus, daß sie Liganden für Ca²⁺- und K⁺-Kanäle sind, als auch daraus, daß sie antioxidative Eigenschaften besitzen. Hieraus kann eine prophylaktische und/oder eine therapeutische Wirkung abgeleitet werden.

Bezüglich der bereits erwähnten Steroid-Antagonisten ist zwischen puren Antagonisten und partiellen Agonisten zu unterscheiden. Ein purer Agonist weist keine intrinsischen antagonistischen Eigenschaften auf und ist für einen speziellen Rezeptor geeignet. Antagonisten, die partiell als Agonisten wirken, sind mindestens in einem Bereich ihres Anwendungsspektrums rezeptorunspezifisch und können je nach Art des Rezeptors unterschiedliche Wirkungen entfalten. Die Wirkung ist somit davon abhängig, im Bereich welchen Organes oder welchen Körperteiles die Wirkung entfaltet wird. Zusätzlich sind auch die Metaboliten der oben erwähnten Substanzen einsetzbar. Eine spezielle Klasse der Steroid-Antagonisten sind die Anti-Androgene, die ebenfalls pure oder partielle Eigenschaften aufweisen können und von denen gleichfalls die Metaboliten verwendbar sind.

Eine weitere Untergruppe der Steroid-Antagonisten bilden die Anti-Östrogene, die ebenfalls mit purem oder partiellem Eigenschaftsspektrum versehen sein können und die diesen zugeordneten Metaboliten und Abkömmlinge. Zentral wirksame Aromatasehemmer gehören zur Gruppe der funktionellen Östrogenantagonisten.

Desweiteren sind als Untergruppe der Steroid-Antagonisten die Anti-Progesterone mit ihren zugeordneten Metaboliten verwendbar, auch wiederum mit purem oder partiellem Eigenschaftsspektrum.

Eine Vielzahl geeigneter Steroid-Antagonisten wird in der Veröffentlichung "Comparative Affinity of Steroidal and Nonsteroidal Antioestrogens, Cholesterol Derivatives and Compounds with a Dialkylamino Side Chain for the Rat Liver Antioestrogen Binding Site" Biochemical Pharmacology, Vol. 43, No. 12, pp. 2511-2518,1992., C.D.M.A. van den Koedijk, C. Vis van Heemst, G.M. Elsendoorn, J.H.H. Thijssen and M.A. Blankenstein, beschrieben. Hervorzuheben sind hier insbesondere die Triphenylethylene Antiöstrogene und die nicht-Triphenylethylene Antiöstrogene. Derartige Substanzen sind insbesondere Tamoxifen, 4-OH-Tamoxifen, 3-OH-Tamoxifen, N-Desmethyltamoxifen, Monophenoltamoxifen, Cyanotamoxifen, Toremifene, 4-OH-Toremifene, N-Desmethyltoremifene, Monophenoltoremifene, Deaminotoremifene, Clomiphene, Nafoxidine, Zindoxifene, Trioxifene, Keoxifene, CB 7432; ICI 164, 384; D 18954; ZK 119.010; D 15414; LS 3360; LS 3348; LS 3347; ORG 31710; RU 486 ORG 31376.

Darüber hinaus können Prednimustine und Estramustine angeführt werden.

Außer den bereits beschriebenen Steroid-Antagonisten können alternativ oder ergänzend Steroid-Agonisten eingesetzt werden, die wiederum als pure Agonisten oder als partielle Antagonisten ausgebildet sein können. Darüber hinaus ist die Verwendung von Androgenen, Östrogenen oder Progesteronen möglich, jeweils mit purem oder partiellem Wirkungsprofil und jeweils unter Einbeziehung der Metaboliten.

Bei der akuten Wirkung des Präparates tritt zunächst eine Wechselwirkung mit den Rezeptoren auf, die eine Änderung der Zellaktivität zur Folge hat und darüber hinaus Zellsubstanzen freisetzt, die eine Trennung des Liganden vom Rezeptor begünstigen. Nach einer entsprechenden Trennung wird der Ligand im Rahmen des normalen Stoffwechsels abgebaut. Zur Aufrechterhaltung oder Wiederauffrischung der Wirkung ist es somit erforderlich, in bestimmten zeitlichen Abständen eine neue Dosis des Präparates zu applizieren.

## Patentansprüche

1. Präparat zur Therapie und Prophylaxe von Demenz-Erkrankungen, insbesondere von Erkrankungen aufgrund von nachlassender Zellaktivität, dadurch gekennzeichnet, daß als Wirkstoff eine Dosis mindestens eines Steroid-Antagonisten enthalten ist.

2. Präparat nach Anspruch 1, dadurch gekennzeichnet, daß der Steroid-Antagonist als ein rezeptorspezifischer purer Antagonist ausgebildet.

3. Präparat nach Anspruch 1, dadurch gekennzeichnet, daß der Steroid-Antagonist mindestens bereichsweise als rezeptorunspezifischer partieller Agonist ausgebildet ist.

4. Präparat nach einem der Ansprüche 1 bis 3, dadurch gekennzeichnet, daß der Wirkstoff als Metabolit des Steroid-Antagonisten ausgebildet ist.

5. Präparat nach Anspruch 1, dadurch gekennzeichnet, daß der Steroid-Antagonist als ein Anti-Androgen ausgebildet ist.

6. Präparat nach Anspruch 5, dadurch gekennzeichnet, daß das Anti-Androgen als ein rezeptorspezifisches pures Anti-Androgen ausgebildet ist.

7. Präparat nach Anspruch 5, dadurch gekennzeichnet, daß das Anti-Androgen mindestens bereichsweise als rezeptorunspezifischer partieller Agonist ausgebildet ist.

8. Präparat nach einem der Ansprüche 5 bis 7, dadurch gekennzeichnet, daß der Wirkstoff als Metabolit des Anti-Androgens ausgebildet ist.

9. Präparat nach Anspruch 1, dadurch gekennzeichnet, daß der Steroid-Antagonist als ein Anti-Östrogen ausgebildet ist.

10. Präparat nach Anspruch 9, dadurch gekennzeichnet, daß das Anti-Östrogen als ein rezeptorspezifisches pures Anti-Östrogen ausgebildet ist.

11. Präparat nach Anspruch 9, dadurch gekennzeichnet, daß das Anti-Östrogen mindestens bereichsweise als rezeptorunspezifischer partieller Agonist ausgebildet ist.

12. Präparat nach einem der Ansprüche 9 bis 11, dadurch gekennzeichnet, daß der Wirkstoff als Metabolit des Anti-Östrogens ausgebildet ist.

13. Präparat nach Anspruch 1, dadurch gekennzeichnet, daß der Steroid-Antagonist als ein Anti-Progesteron ausgebildet ist.

14. Präparat nach Anspruch 13, dadurch gekennzeichnet, daß das Anti-Progesteron als ein rezeptorspezifisches pures Anti-Progesteron ausgebildet ist.

15. Präparat nach Anspruch 13, dadurch gekennzeichnet, daß das Anti-Progesteron mindestens bereichsweise als rezeptorunspezifischer partieller Agonist ausgebildet ist.

16. Präparat nach einem der Ansprüche 13 bis 15, dadurch gekennzeichnet, daß der Wirkstoff als Metabolit des Anti-Progesterons ausgebildet ist.

17. Präparat zur Therapie und Prophylaxe von Demenz-Erkrankungen, insbesondere von Erkrankungen aufgrund von nachlassender Zellaktivität, dadurch gekennzeichnet, daß als Wirkstoff eine Dosis mindestens eines Steroid-Agonisten enthalten ist und daß der Steroid-Agonist mindestens bereichsweise als rezeptorunspezifischer partieller Antagonist ausgebildet ist.

18. Präparat nach Anspruch 17, dadurch gekennzeichnet, daß der Wirkstoff als Metabolit des Steroid-Agonisten ausgebildet ist.

19. Präparat nach Anspruch 17, dadurch gekennzeichnet, daß der Steroid-Agonist als ein Androgen ausgebildet ist und daß das Androgen mindestens bereichsweise als rezeptorunspezifisches partielles Anti-Androgen ausgebildet ist.

20. Präparat nach Anspruch 19, dadurch gekennzeichnet, daß der Wirkstoff als Metabolit des Androgens ausgebildet ist.

21. Präparat nach Anspruch 17, dadurch gekennzeichnet, daß der Steroid-Agonist als ein Östrogen ausgebildet ist und daß das Östrogen mindestens bereichsweise als rezeptorunspezifisches partielles Anti-Östrogen ausgebildet ist.

22. Präparat nach Anspruch 21, dadurch gekennzeichnet, daß der Wirkstoff als Metabolit des Östrogens ausgebildet ist.

23. Präparat nach Anspruch 17, dadurch gekennzeichnet, daß der Steroid-Agonist als ein Progesteron ausgebildet ist und daß das Progesteron mindestens bereichsweise als rezeptorunspezifisches partielles Anti-Progesteron ausgebildet ist.

24. Präparat nach Anspruch 23, dadurch gekennzeichnet, daß der Wirkstoff als Metabolit des Progesterons ausgebildet ist.
